# EUROPEAN PATENT APPLICATION

(11) **EP 3 556 289 A1**
(43) Date of publication of application: **23.10.2019**
(21) Application number: 19170248.9
(22) Date of filing: 18.04.2019
(51) Int. Cl.: A61B 5/11, A61B 5/024

(54) **WEARABLE DEVICE**

(30) Priority: 18.04.2018 NL 2020786
(71) Applicant: mQare IP B.V., 2201 BB Noordwijk (NL)
(72) Inventor: Vrolijk, Reinier, 2596 XA Den Haag (NL); Olie, Ronald, 2201 DM Noordwijk (NL)
(74) Representative: V.O.

(57) **Abstract**

The present invention relates to a smart wearable device enabling the measurement of vital parameters indicative of health status. There is further provided a monitoring and alert system of health status capable of processing measurement data sent by a smart wearable device. The monitoring and alert system is further capable of triggering alerts based on measurement values performed by the smart wearable device. Further provided is a method for monitoring a health status by means of a smart wearable device and a monitoring and alert system.

## Description

### TECHNICAL FIELD

The invention relates to a smart wearable device, such as a wrist band, arm band, a watch or a bracelet that may be worn on the upper arm, lower arm or other body part. The invention further relates to a monitoring and alert system including a smart wearable device, and a method of monitoring and providing alerts based on measurements of physiological parameters enabled through a smart wearable device.

### BACKGROUND

With an aging population and increasing costs of healthcare, there is a desire to have elderly people live in their own homes as long as possible. There is a further desire to minimise surveillance by direct human presence, and replace this by remote monitoring via technological non-intrusive instruments. Mobile phones and in particular smart phones may provide suitable functionalities to achieve this, but are not always carried along everywhere.

Other suitable devices may be wearables, such as sport watches or the like. These smart wearables have been developed to be worn on the wrist, chest or other parts of the body to provide a user with a plurality of functions beyond the basic indication of time as enabled by ordinary wrist watches. GPS sensors and internet connectivity allow e.g. runners to keep track of their running courses and distances, performance and progression.

However, smart watches may have a look and feel not to everybody's liking. It may be too flashy, bulky or eye-catching. And it may require care and attention in order to keep working, such as daily charging of the battery. Furthermore, the digital user interface may not be easily handled by elderly people. Therefore, elderly people may desire devices that are less conspicuous and that may work to a large extent unattended; or at least for a substantial period.

In particular, elderly may be hindered in their mobility or at least may have anxieties about their mobility, which in turn influences their confidence. For them, a re-assurance that help will be available when needed to rely upon may improve their quality of life. Hence, there still is a need to make further functionalities available that address the needs and desires of other user groups in other form factors.

### SUMMARY

It is an object of the invention to provide a smart wearable device that provides additional functionalities addressing the above-mentioned disadvantages.

Accordingly, there is provided a smart wearable device enabling the measurement of vital parameters indicative of health status. These vital parameters include blood pressure, heart rate i.e. cardiac rhythm, respiration / breathing rate, and / or a change in state of consciousness.

There is further provided a monitoring and alert system of health status capable of processing measurement data by a smart wearable device.

There is further provided a monitoring and alert system of health status capable of triggering alerts based on measurement values performed by the smart wearable device.

There is further provided a method for monitoring a health status by means of a smart wearable device and a monitoring and alert system.

In one aspect the smart wearable device comprises means for determining location / localisation information, communication means for exchanging data over a communication network, and vital parameter determination means, comprising pulse determination means for determining a heart rate. The smart wearable further comprises a memory module for storing configuration settings and vital parameter measurement data from the vital parameter determination means, and a control unit. The control unit is arranged for controlling the vital parameter determination means for performing measurements in accordance with the configuration settings, controlling the memory module for storing measurement data in accordance with the configuration settings, and controlling the communication means for transmitting measurement data in accordance with the configuration settings. The control unit is further arranged for adjusting the configuration settings in response to receiving a configuration setting update, and/or detecting an outbound condition of vital parameter measurement data.

In one aspect the smart wearable device comprises one or more alarm buttons and one or more recall / neutralisation buttons.

In another aspect, the smart wearable device further comprises means for detecting at least one of the following parameters: blood oxygen saturation, respiratory rate, and / or skin temperature.

In yet another aspect, the smart wearable device may comprise means for a pedometer

In one aspect, the alerting system comprises a smart wearable device, a communication server and an online accessible interface application. The online accessible interface application will allow access to confided users.

In another aspect, the alerting system may further comprise a mobile application i.e. software program for a mobile phone.

In healthcare and medical environments, the monitoring of vital parameters of patients is known. These vital parameters include blood pressure, heart rate i.e. cardiac rhythm, oxygen saturation, respiration / breathing rate, and / or a change in state of consciousness. Each of these vital parameters is associated with certain triggers and / or thresholds which provide a basis for (Modified) Early Warning Score (MEWS or EWS) criteria.

A Modified Early Warning Score, MEWS, has further been developed. This MEWS appoints certain scores to the crossing of the thresholds for each vital parameter, the aggregation of which provides a total score as an indication of the need for increased monitoring and / or preventive measures.

Hence, the monitoring of these parameters allows an early assessment of vital deterioration, which increases the chances of timely response for addressing life threatening medical situations.

However, these assessments are based on static trigger points, single valued thresholds that determine whether a measurement point is outbound of a range of normal / regular expected values i.e. the relevant value of the parameter as such value has been measured, calculated or otherwise determined is outside a range, which range may be predetermined and/or determined ad hoc. So, this basically provides a statistic assessment of parameters.

Moreover, the initiative to start monitoring such parameters is often only taken after incidents have occurred or once a patient is already hospitalised.

Research has shown that prior to a deterioration of health status that might be leading up to cardiac arrest, 60 - 70% of such patients (Schein 1990) experience physical irregularities and / or instabilities. These may include problems relating to respiratory system or other abnormal conditions.

The smart wearable device, the monitoring and alert system and the method according to the invention allow a dynamical assessment of health status. As these enable monitoring development of vital parameters in time, prior to the static assessment of health status. Thus, a more dynamic, time-dependent, assessment of vital parameters.

Moreover, the smart wearable device, the monitoring and alert system and the method allow collecting of health status anonymised data that enables big data analysis. Which may result in the discovery of medical / vital time-dependent patterns indicative of an upcoming deterioration of health status and consequently may provide further early warning signals. The collected data may also be validated and/or labelled.

Hence, the combined system not only enables population analysis, allowing to collect and process larger amounts of data and thereby discovering additional patterns more quickly; this in contrast to monitoring isolated individuals. It also allows to tailor to the individual and adapt certain thresholds to the specific user of the wearable device, e.g. when certain parameter deviations are known in advance. Not merely applying known patterns derived from group analysis to an isolated individual, but tailoring to a specific individual.

### BRIEF DESCRIPTION OF THE DRAWINGS

Figure 1 illustrates an example of a smart wearable device;
Figure 2 shows a perspective bottom view of part of smart wearable device;
Figure 3 shows a cross-sectional view of a smart wearable device;
Figure 4 shows an exploded view of a modular smart wearable device;
Figure 5 illustrates schematically a system for monitoring and alert; and
Figure 6 illustrates a method for monitoring and alert.

### DETAILED DESCRIPTION

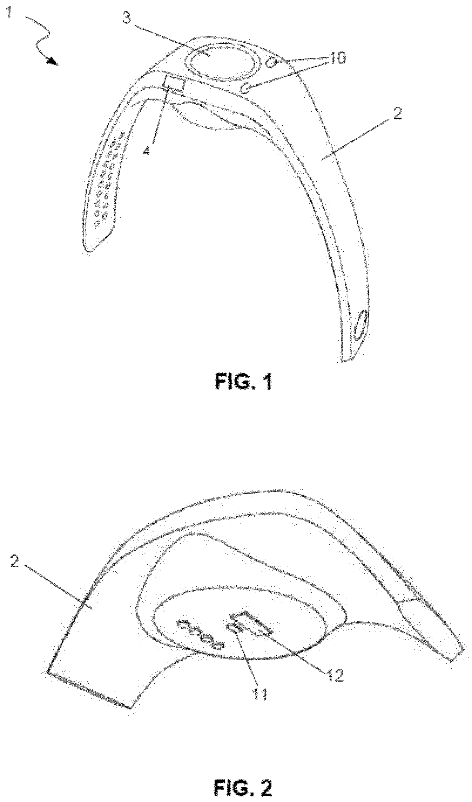

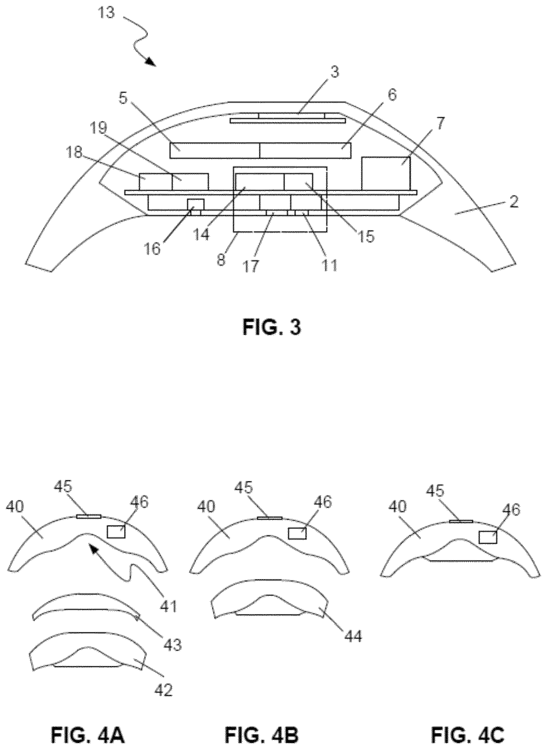

Fig. 1 shows a smart wearable device 1, in this example a wrist band 2, having an alarm button 3 and a neutralisation button 4 having two touch portions on opposite sides of the wrist band 2. The smart wearable device 1 further has internal components located within, as shown in the example of Fig. 3, including localisation means 5 for determining a location, and communication means 6 for exchanging data over a communication network. The smart wearable further has fall detection means 7 for detecting a fall, pulse determination means 8 for determining a heart rate. The wearable device 1 of Fig. 1 further has multiple lights 10, in this example LEDs, for providing visual signals.

The alarm button 3 may be pushed for generating a signal activating an alarm. In response to the activated alarm, one or more of the LEDS 10 will start flashing, and an alert message will be sent via the communication means 6. In some examples, the wearable 1 may further be equipped with a buzzer for providing auditive signals in response to activation of an alarm.

In this example, the neutralisation button 4 has two touch portions which need to be pushed together simultaneously in order to generate a neutralisation signal. The neutralisation signal will reset the alarm, if it was active, and send a reset message indicating that no alarming situation is present. The use of a two touch portion button may prevent unintended or accidental activation of the neutralisation signal. In other examples, the neutralisation button 4 may be a single piece button. In yet other embodiments, it may be that the alarm button acts as neutralisation button when pressed multiple times or e.g. for a duration of several seconds.

In the example of Fig. 1, the localisation means 5 for determining a location includes a GNSS (Global Navigation Satelite System) sensor as known in the art. In other examples, the localisation means 5 may include other suitable means for determining the location, such as difference(s) in signal strength or transmission time(s), as known in the art. The communication means 6 for exchanging data over communication network are arranged for Narrow Band - Internet of Things, NB-IoT in short. These means include an antenna, transceiver and further required components as known in the art. In other examples, the communication means may be arranged for other network standards; preferably Low Power Wide Area (LWPA or LWPAN) such as for example SigFox or LoRa, and / or LTE-M Network. In yet other examples, the communication means 6 may further provide for Bluetooth connectivity.

The fall detection means 7 may include one or more accelerometers for detecting a fall, in this example one 3-dimensional accelerometer. The accelerometer will sense acceleration from which the movement in three distinct directions may be derived. The main function is to detect a fall, which may be sensed by a continuous acceleration in line with the earth's gravitational field. However, various other motion algorithms may be applied.

In addition to accelerometers, further inertial and/or magnetic sensors may be used to obtain 6 Degrees of freedom, 6DOF, measurement capability to measure e.g. tilt. These may be further expanded with a 3-axis gyroscope sensors to obtain a 9DOF, sometimes referred to as gyro-stabilized measurements. The measurements established with these sensors may be used to determine various types of activities, such as walking, sitting, running, lying down, etc.

Moreover, in other examples the one or more accelerometers of the fall detection means 7 may be used as pedometer for step counting and / or for sleep detection by employing additional motion algorithms used for the signal processing.

Turning to Fig. 2, the location of the sensor means are shown in more detail. The pulse determination means 8 include a light source 11, in this example one for emitting green light. The pulse determination means 8 further include a light sensitive photodiode 12 for sensing light. When the smart wearable is worn by a user, light emitted from the light source 11 will travel into the skin and partially be reflected, a process known as backscattering. A fraction, usually only a small fraction, of the light injected into the skin will be received by the photodiode 12. This combination of light source 11 and photodiode 12 taking advantage of backscattering is also referred tot as performing Photoplethysmogram i.e. PPG measurements. In general, a Photoplethysmogram (PPG) is an in vivo optically obtained Plethysmogram, which may provide an approximation of the volumetric blood pulsation from a part of the body. The pulsation of the blood influences the backscattering of the light travelling into the skin, and accordingly the pattern of the received light provides a measure for the pulsation which corresponds to a heart rate. Alternatively or additionally, electrical sensors may be used for determining pulse and/or heart rate.

Next to determining a heart rate, the same light backscattering principle may also be employed for determining a level of oxygen saturation of the blood, also referred to as pulse oximetry. The level of oxygen saturation is a measure for the amount of oxygen that is bound to haemoglobin, in short oxyhaemoglobin, and the amount of haemoglobin without oxygen. Oxyhaemoglobin absorbs infra-red light more than haemoglobin, whereas haemoglobin absorbs red light more than oxyhaemoglobin. Thus, injecting red light and infrared light into the skin will experience the same backscattering and may again be received by a photodiode. From these measurements, also referred to as SpO₂ measurements, the saturation level may be determined. Under normal circumstances this will be around 95% or more. Another parameter that may determined from these measurements is a rate of respiration. In other embodiments, the rate of respiration may be determined from a heart rhythm variation.

In order to improve the sensitivity for picking up the light reflected through backscattering, for each specific light emitted, viz. green, red, and infra-red, three photodiodes may be implemented, each being dedicated to one specific light source. So, one photodiode for green light, one for red light and one for infra-red light. In other embodiments, a single photodiode may be used for sensing both red and infra-red light. In a further embodiment, even one photodiode may be used for sensing all: green, red and infra-red light.

In yet other embodiments, measurements may be based on a broad spectrum of joint green-red light on the one hand and infra-red light on the other hand. As in the light spectrum green and red may be considered as close, respectively around 530 nm and 650 nm, versus infra-red at about 940 nm.

Apart from PPG based sensors, also sensors based on Bioelectrical Impedance Vector Analysis, BIVA in short, may be applied. And furthermore, also sensors based on Bioelectrical Impedance Analysis BIA or Galvanic Skin Resistance GSR. These in addition to or instead of PPG based sensors. Or any combination thereof.

Turning to Fig. 3, another example of a smart wearable device 13 is shown. In addition to the example of Fig. 1, the wearable further includes detection means for detecting at least one or more of the following parameters: oxygen saturation of the artery bloodstream 14, respiration or breathing rhythm 15, and / or skin temperature 16 indicative of body temperature. In this example, no alarm button or neutralisation button are provided.

In this example, means for detecting the oxygen saturation 14 and the respiration 15 are incorporated in the pulse determination means 8. Thereto, the pulse determination means now include three light sources, one for emitting green light, one for emitting red light and one for emitting infra-red light. A similar photodiode 17 as in described in relation to Fig. 1 is also included, which next to green light is now also configured to be sensitive for red and infra-red light.

In general, to further enhance the sensitivity of the pulse determination means 8, the portion of the wearable device where these are located may be surrounded by an upstanding ridge, which provides an enclosure when the wearable is worn around the wrist or arm portion. In other embodiments, instead of or in addition to the upstanding ridge, there may be a protruding portion pushing against the part of the wrist or arm where contact is made.

The means for detecting skin temperature 16 indicative of body temperature include a temperature sensor that measures temperature close to or, more preferably, on the skin. From the measured skin temperature, the body temperature may be derived, as the temperature profile of the body temperature at the body part location of measurement corresponds to a gradient starting within an inner core of the body part and diminishing outwards to the outer skin.

The smart wearable device 1, 13 may further include a memory module 18 for storing an executable program, measurement data and / or configuration settings. And processing means 19 for executing the executable program. The executable program will include all processing instructions required for providing the functionalities of the smart wearable.

Referring to Figs. 4A - 4C, a modular assembly of the smart wearable according to the invention is shown. A wristband wearing portion 40 having a cavity 41 is shown in Fig. 4A, together with a sensor carrier portion 42 and a cover portion 43.

The sensor carrier portion 42 has a form-factor dimensioned to form-fit in the cavity 42 of the wearing portion 40. Thus; the cavity 41 of the wrist band may accommodate the sensor carrier portion 42. Furthermore, the sensor carrier portion 42 also has a cavity. The cover portion 43 has a form-factor dimensioned to close-of the cavity of the sensor carrier portion 42. Thus; the cavity of the sensor carrier portion 42 may accommodate the cover portion 43 to snugly fit on top.

Accordingly, as shown in Fig. 4B, the cover portion 43 closes off the sensor carrier portion 42 as a lid. And the assembly 44 of cover 43 and sensor carrier 42 may be accommodated by the cavity 41 of the wearing 40, as shown in Fig. 4A.

As will be understood, the various portions may also be implemented in less parts or as a single integral piece.

Though in the example of Fig.4A-C, embodied as wrist band, it should be understood that an arm band that may be e.g. worn at the upper arm or the lower arm is also contemplated. Furthermore, other parts of the body such as leg, ankle or neck may also be considered as suitable location.

Further shown in Figs. 4A - 4C is that the one or more alert buttons 45 and one or more neutralisation 46 buttons are positioned on the wristband wearing portion 40. The localisation means for detecting a location, the communication means for exchanging data over a communication network, and the fall detection means for detecting a fall are positioned in the sensor carrier portion 42. Also the pulse determination means are positioned in sensor carrier portion.

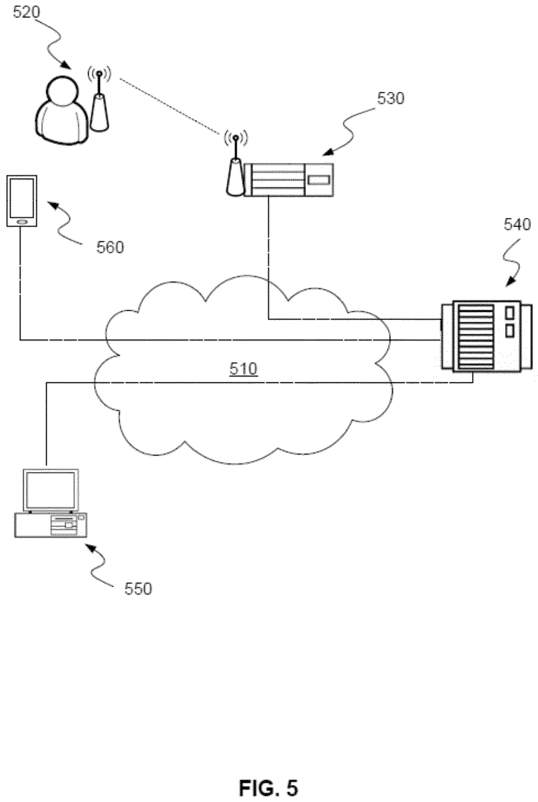

Shown in Fig. 5 is an example of a monitoring and alert system 500 of health status. The system 500 is capable of processing alerts sent by the smart wearable device. The system 500 is further capable of triggering alerts based on measurements performed by the smart wearable device.

The system 500 allows not only alarming medical personnel in case of one or more vital parameters showing outbound values assessed with respect to a static health status model. It also allows alarming medical personnel in case of vital parameters showing outbound values assessed with respect to a dynamic health status model. Outbound values are classified as such by comparison to values relating to regular circumstances during resting, walking, sporting, sleeping, etc.

The monitoring and alert system 500 includes one or more smart wearable devices as described above which in Fig. 5 is indicated by user access point 520. The system 500 further includes a back-end server infrastructure 540 and an online interface application accessible by a computer 550 or mobile device 560. Communication within the system is arranged through a communication network 510 provided by such service providers which includes local network access points 530.

The server infrastructure 540 provides storage, processing and analysis of measurement data acquired by the smart wearable 520. The smart wearable 520 sends the acquired measurement data over the communication network 510, 530 to the server infrastructure 540 . Based on the results of the processing and analysis performed by the server infrastructure 540 alerts may be generated. The results and generated alerts will be available to the central helpdesk. Confided users of the interface application may initiate actions based on the provided results and alerts. This may include alerting relatives of the user of the smart wearable, medical personnel, or other persons that the user has indicated as to be informed. Moreover, the system 500 may be arranged to automatically initiate sending alert messages to indicated persons.

The measurement data along with the processing and analysis results on the server infra-structure 540 of a particular user of a smart wearable 520 may also be available to that user by means of the online accessible interface application, as a kind of dashboard. The dashboard may be a web based application, it may be a dedicated mobile phone application or other software program allowing access to the users' own data.

Besides the user of the smart wearable him/herself, other persons indicated by the user may also be provided access to the measurement data available on the server infra-structure. Such indicated users form a group of confided users that are provided with user credentials, such as a login and password combination or other known means for arranging secure access to online software applications.

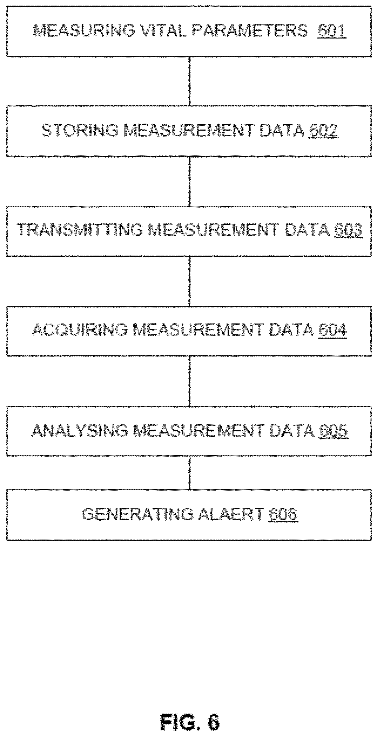

Referring to Fig. 6, an example is shown of a method for monitoring a health status by means of a smart wearable device and monitoring and alert system. In order to acquire data at certain predetermined time intervals, the method includes the smart wearable periodically measuring 601 vital parameters. Preferably, this is performed a number times per second. Then temporarily storing 602 the measured vital parameters in a memory on the smart wearable device as vital parameter measurement data. And transmitting 603 the vital parameter measurement data to a back-end server infrastructure.

The method further includes acquiring 604 measurement data of vital parameters from the smart wearable device by the server infrastructure. Analysing 605 the measurement data for outbound conditions of vital parameter values. And in case of outbound conditions, generating an alert 606 for the outbound vital parameter, i.e. if the parameter is outside a particular range or above or below a particular value.

In addition to analysing measurement data 605 for outbound conditions, the analysing step 605 may include analysing for irregular patterns. In turn, an alert may also be generated 606 for a detected irregular pattern.

Table 1 below shows examples of vital parameter values for assessing an outbound condition. In turn, simultaneous occurrence of changes in vital parameters may form a regular pattern; as for example when running the cardiac rhythm and respiration will increase, or when sleeping these will decrease. Hence, regular patterns are known and / or may be obtained over time; which will provide indications of when a non-regular pattern may be classified as irregular.

**Table 1.**

| **Vital parameter** | **Lower threshold** | **Regular range** | **Upper threshold** |
|---|---|---|---|
| Oxygen saturation | ≤ 85 | ≥ 95 | n.a. |
| Respiration | ≤ 9 | 9 - 14 | ≥ 30 |
| Cardiac rhythm | ≤ 40 | 51 - 100 | ≥ 130 |
| Blood pressure | ≤ 70 | 101 - 180 | ≥ 220 |
| Temperature | ≤ 35 | 36.0 - 37.5 | ≥ 38.5 |

Each time measurement data has been transmitted by the smart wearable device and is acquired by the back-end server infrastructure, the smart wearable device will start to free up partially or clear in full the storage capacity of the memory. This is preferably performed in response to receiving an acknowledgment from the server infrastructure that all data has been acquired successfully.

In order to make sure that the storage capacity of the memory available on the smart wearable device is not surpassed, the acquiring of measurement data 604 by the server infrastructure is performed a few times per day. Or at least so many times as to prevent the smart wearable not able to store additional measurement data. Under circumstances wherein network availability, storage and battery capacity are not constrained, acquisition of data may be performed every five or ten minutes, every two or four hours, or another freely chosen time interval. In turn, the smart wearable may be arranged to free up some partial memory in case the maximum capacity is nearly reached.

As the transmitting 603 of measurement data may interfere with other operational tasks of the smart wearable, such as periodic measurement of vital parameters or generating an alert, the amount of time required or allowed for transmitting the data is preferably kept to a minimum. That means that the timeout for communication between smart wearable and server infrastructure is reduced as much as possible to prevent interference with measurement of parameters.

Preferably, parameter data measurements are performed at coherent time instances and likewise jointly transmitted. For example, every two or four hours all measurement data is transmitted by the device 1 and acquired by the server infrastructure.

Furthermore, the smart wearable device is intended to be an inconspicuous small wearable device, that does not provide any hinderance to its' user so that it will be worn all day without nuisance. This implies small size and a small battery with limited power capacity. This also has an influence on the choice of communication network, being preferably a low power network, such as SigFox, LoRa, LTE-M or NB-IoT. The choice of network in turn determines that bandwidth and transmission speed will be limited. The small size of the smart wearable device also implies limited storage capacity requiring a minimum of power.

The measurement data may be stored uncompressed when no constraints on network availability and/or storage or battery capacity are presented. Nevertheless, it may be understood that storing data in a compressed manner. In another manner, data indicative of stable or regular conditions may be represented in a condensed manner, such as single reference to a previous measurement or as non-deviating from prior measurements and/or expected values or as compliant with a particular activity pattern.

In other examples, merely minimum and maximum values or ranges may be stored. Also histograms and/or representations thereof may be stored. Over time, from selected periods certain measurement data may removed in order to preserve just an indicative representation of measured parameter values. Such as an average value of a particular parameter, or an index indicating a predetermined activity pattern referring to a set of coherent parameter values. Such as heart rate, temperature, and oxygen saturation indicative of walking.

Accordingly, optimum needs to be found in the amount of data that is stored, the amount of data that is transmitted at a time, and the number of times that data is transmitted. As each timeout for transmittal may interfere with the measurement operation of the smart wearable.

Moreover, in case outbound conditions or irregular patterns are observed, an increase in the intensity of monitoring may be desired; such as increasing the periodic measurements or increasing the number of transmittals of measurement data per day, per hour, per minute or any other suitable time unit. Which requires a re-assessment of the optimum of the above-mentioned variables: amount of data stored, amount of data transmitted and number of times of transmittal.

In order to address the above, the method may further include adjusting settings for measuring vital parameters, for selecting which data to store and for transmitting data. Adjustments for transmitting data may include increasing the number of times data is transmitted, and/ or selecting or deselecting data for particular vital parameters to be transmitted or not.

The method may further include adjusting the type of measurements performed. Such as for example, stopping temperature measurement in order to increase measurements of cardiac rhythm. Or other trade-offs or deviations in measurement to enhance the efficacy of the monitoring of vital parameters for a particular situation. In this manner, the method of monitoring may be tailored to the specific user of the smart wearable.

More importantly, the method allows to adjust the parameters to be measured and stored in dependence of previous measurements, in dependence of local circumstances or predicaments, such as an outbreak of influenza, or in dependence of periodical events, such as annual winter colds.

In one embodiment particularly suited for supporting the method disclosed above, the smart wearable device for monitoring a health status includes localisation means (5) for detecting location, communication means (6) for exchanging data over the communication network and vital parameter determination means which include pulse determination means (8) for determining the heart rate. The smart wearable device of this example further includes a memory module (18) for storing configuration settings and vital parameter measurement data from the vital parameter determination means, and a control unit (19). The control unit (19) is arranged for controlling the vital parameter determination means for performing measurements in accordance with the configuration settings, for controlling the memory module for storing measurement data in accordance with the configuration settings, and for controlling the communication means for transmitting measurement data in accordance with the configuration settings. To enable the method as disclosed the control unit is further arranged for adjusting the configuration settings in response to receiving a configuration setting update. And /or for adjusting the configuration settings in response to detecting an outbound condition of vital parameter measurement data.

In this example, the configuration settings of the smart wearable device include time interval settings for measurements, storage and transmissions. These configuration settings thereto include a first set of time intervals for periodically measuring (601) vital parameters, a second set of time intervals for temporarily storing (602) the measured vital parameters on the smart wearable device as vital parameter measurement data. And a third set of time intervals for transmitting (603) the vital parameter measurement data to a monitoring system (540). The configuration settings may further include a set of vital parameter values defining outbound conditions of vital parameter measurement data.

The sets of intervals may each comprise one or more intervals of time intervals. Of one or each set of intervals, an appropriate interval may be selected, based on at least one of a value of at least one vital parameter, quality of network condition, location of the device 1 as determined by the device 1, conditions of the environment at the determined location, like temperature, humidity, precipitation, other weather conditions, information on potential bacterial, fungal or viral infections at the determined location, other, or a combination thereof.

Moreover, in case a particular outbreak of a bacterial nature or other health-threatening circumstances, the monitoring method may be adjusted likewise. For example, each year in winter new types of influenza become apparent and the measurement of e.g. temperature may become more relevant for indicating the health status of the user.

As another example, there might be the announcement of an outbreak of legionella incident at a particular location. If according to the location history, if available, it is judged that the user has been near the location of the outbreak, appropriate adjustments may be made to the method of monitoring. Such could be done through the online application accessible for confided users, which for example may include the personal doctor of the user.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

Furthermore, although exemplary embodiments have been described above in some exemplary combination of components and/or functions, it should be appreciated that, alternative embodiments may be provided by different combinations of members and/or functions without departing from the scope of the present disclosure. In addition, it is specifically contemplated that a particular feature described, either individually or as part of an embodiment, can be combined with other individually described features, or parts of other embodiments.

## Claims

1. Smart wearable device for monitoring a health status, comprising:
localisation means (5) for detecting a location;
communication means (6) for exchanging data over a communication network;
vital parameter determination means, comprising pulse determination means (8) for determining a heart rate;
a memory module (18) for storing configuration settings and vital parameter measurement data from the vital parameter determination means; and
a control unit (19);
wherein the control unit is arranged for:
controlling the vital parameter determination means for performing measurements in accordance with the configuration settings;
controlling the memory module for storing measurement data in accordance with the configuration settings;
controlling the communication means for transmitting measurement data in accordance with the configuration settings; and
adjusting the configuration settings in response to:
receiving a configuration setting update; and/or
detecting an outbound condition of vital parameter measurement data.

2. Smart wearable device according to claim 1, wherein the configuration settings comprise:
a first set of time intervals; for periodically measuring (601) vital parameters;
a second set of time intervals for temporarily storing (602) the measured vital parameters on the smart wearable device as vital parameter measurement data;
a third set of time intervals for transmitting (603) the vital parameter measurement data to a monitoring system (540);
a set of vital parameter values defining outbound conditions of vital parameter measurement data.

3. Smart wearable device according to claim 1 or 2, further comprising:
fall detection means (7) for detecting a fall; and
the vital parameter determination means further comprising determination means for determining at least one of the following parameters:
oxygen saturation of artery bloodstream (14);
breathing rhythm (15);
skin temperature (16) indicative of body temperature.

4. Smart wearable device according to any of claims 1 - 3, further comprising:
one or more alarm buttons (3); and / or
one or more neutralisation buttons (4).

5. Smart wearable device according to any of the preceding claims, further comprising:
a memory module (18) for storing a computer executable program, measurement data and / or configuration settings; and
wherein the control unit comprises processing means (19) for executing the executable program.

6. Smart wearable device according to any of the preceding claims, further comprising:
a wearing portion (40) having a cavity (41);
a sensor carrier portion (42) having a cavity and having a form-factor dimensioned to form-fit in the cavity (41) of the wearing portion (40); and
a cover portion (43) having a form-factor dimensioned to close-off the cavity of the sensor carrier portion (42).

7. Smart wearable device according to claim 6, wherein:
the one or more alarm buttons (3) and one or more neutralisation buttons (4) are positioned on the wearing portion (40);
the localisation means (5) for detecting a location, the communication means (6) for exchanging data over a communication network, the fall detection means (7) for detecting a fall, and the pulse determination means (8) are positioned in the sensor carrier portion (42).

8. A monitoring and alert system, comprising:
one or more smart wearable devices (1, 13, 520) according to any of claims 1 - 7;
a server infrastructure (540);
an online interface application (550).

9. A monitoring and alert system according to claim 8, further comprising:
a mobile application executable on a smart phone (560).

10. A method for monitoring a health status by means of a smart wearable device (1, 13, 520) according to any of claims 1 - 7 and a monitoring and alert system (500) according to claim 8 or 9, comprising:
acquiring (604) measurement data of vital parameters from the smart wearable device (1, 13, 520);
analysing (605) the measurement data for irregular patterns of vital parameter values;
generating (606) an alert for a detected irregular pattern.

11. A method according to claim 10, further comprising:
analysing the measurement data for outbound conditions of vital parameter values;
generating an alert for the outbound vital parameter.

12. A method according to claim 10 or 11, further comprising:
the smart wearable device (1, 13, 520) periodically measuring (601) vital parameters;
temporarily storing (602) the measured vital parameters on the smart wearable device as vital parameter measurement data;
transmitting (603) the vital parameter measurement data to the server infrastructure (540).

13. A method according to any of claims 10 - 12, further comprising:
adjusting settings for measuring vital parameters; and / or
adjusting selection of measurement data to store; and / or
adjusting settings for transmitting vital parameter measurement data.

14. A method according to claim 12 or 13, wherein adjusting settings for transmitting vital parameter measurement data comprises:
increasing the number of times data is transmitted; and / or
selecting and/or deselecting measurement data of particular vital parameters to be transmitted.

15. A method according to any of claims 10 - 14, further comprising:
adjusting for which vital parameters measurements are performed.
